# EUROPEAN PATENT APPLICATION

(11) **EP 4 159 707 A1**
(43) Date of publication of application: **05.04.2023**
(21) Application number: 21812400.6
(22) Date of filing: 19.05.2021
(51) Int. Cl.: C07C 19/08, C07C 17/35, C07C 17/361, C07B 61/00

(54) **METHOD FOR PRODUCING MONOFLUOROMETHANE**

(30) Priority: 29.05.2020 JP 2020094771
(71) Applicant: Zeon Corporation, Tokyo 100-8246 (JP)
(72) Inventor: MATSUURA Go, Tokyo 100-8246 (JP)
(74) Representative: Beckmann, Claus
(86) International application number: PCT/JP2021/019064
(87) International publication number: WO 2021/241371

(57) **Abstract**

Provided is a production method that enables production of monofluoromethane by a gas phase flow method without using a catalyst. The method of producing mono fluoromethane includes causing electrical discharge of a feedstock gas containing a fluorine-containing inorganic compound, a compound represented by formula 1: CH₃-R (R is a hydrogen atom, a chlorine atom, a bromine atom, an iodine atom, or an organic group other than a hydrocarbon group), and an inert gas, while in a continuous flow state, and then causing continuous release to outside of an electrical discharge zone.

## Description

### TECHNICAL FIELD

The present disclosure relates to a method of producing monofluoromethane.

### BACKGROUND

Monofluoromethane is widely used in applications such as for an etching gas in microfabrication of semiconductors.

In a known method of producing monofluoromethane, methyl chloride (CH₃Cl) and hydrogen fluoride are reacted in a gas phase in the presence of a fluorination catalyst to obtain a mixed gas containing monofluoromethane, and then monofluoromethane is separated and purified from this mixed gas.

### CITATION LIST

### Patent Literature

PTL 1: JP2006-111611A

### SUMMARY

### (Technical Problem)

However, in the method of producing monofluoromethane described above, production of a fluorination catalyst has been a significant burden, and continuous production has been difficult due to reduction of yield accompanying reduction of catalyst activity, for example.

Accordingly, an object of the present disclosure is to provide a production method that enables production of monofluoromethane by a gas phase flow method without using a catalyst.

The inventor conducted diligent studies to achieve the object set forth above and discovered that monofluoromethane can be obtained by causing electrical discharge of a feedstock gas while in a continuous flow state and then causing continuous release to outside of the electrical discharge zone. In this manner, the inventor completed the present disclosure.

The present disclosure aims to advantageously solve the problem set forth above and relates to a method of producing monofluoromethane that comprises causing electrical discharge of a feedstock gas containing: a fluorine-containing inorganic compound; a compound represented by formula 1: CH₃-R, where R is a hydrogen atom, a chlorine atom, a bromine atom, an iodine atom, or an organic group other than a hydrocarbon group; and an inert gas, while in a continuous flow state, and then causing continuous release to outside of an electrical discharge zone.

Hereinafter, monofluoromethane is also referred to as a "target substance".

The term "inorganic compound" in "fluorine-containing inorganic compound" means a compound that does not include a carbon atom or a compound that includes one carbon atom and does not include a hydrogen atom.

In the presently disclosed method of producing monofluoromethane, the feedstock gas is converted to a reaction gas containing a radical that is a precursor for monofluoromethane through electrical discharge of the feedstock gas while in a continuous flow state, and then monofluoromethane is produced through continuous release to outside of the electrical discharge zone. In this manner, the present disclosure enables production of monofluoromethane by a gas phase flow method without using a catalyst.

In the presently disclosed method of producing monofluoromethane, the fluorine-containing inorganic compound is preferably one or more selected from the group consisting of SF₄, SF₆, SOF₂, SO₂F₂, HF, NF₃, CF₄, BF₃, and SiF₄. These fluorine-containing inorganic compounds enable simple generation of an active species of a simple substance of fluorine during electrical discharge.

In the presently disclosed method of producing monofluoromethane, the inert gas is preferably one or more selected from the group consisting of N₂ and Ar.

In the presently disclosed method of producing monofluoromethane, total proportional content of the fluorine-containing inorganic compound and the compound represented by formula 1 in the feedstock gas is preferably not less than 1 volume% and not more than 85 volume%. When the total proportional content of the fluorine-containing inorganic compound and the compound of formula 1 is within the range set forth above, this enables efficient production of monofluoromethane, which is the target substance.

In the presently disclosed method of producing monofluoromethane, a volume ratio of the fluorine-containing inorganic compound relative to the compound represented by formula 1 is preferably 0.8 or more. When the volume ratio of the fluorine-containing inorganic compound relative to the compound of formula 1 is not less than the lower limit set forth above, production of hydrocarbon side products can be sufficiently inhibited.

Moreover, the volume ratio of the fluorine-containing inorganic compound relative to the compound represented by formula 1 in the presently disclosed method of producing monofluoromethane is preferably 1.8 or more.

### (Advantageous Effect)

According to the present disclosure, it is possible to produce monofluoromethane by a gas phase flow method without using a catalyst. The presently disclosed production method makes it possible to avoid reduction of yield caused by reduction of catalyst activity and enables continuous production of monofluoromethane.

The following provides a detailed description of embodiments of the present disclosure.

### [Fluorine-containing inorganic compound]

The fluorine-containing inorganic compound may be any inorganic compound that includes at least one fluorine atom, with the number of fluorine atoms normally being 8 or less.

The fluorine-containing inorganic compound may be SF₄, SF₆, SOF₂, SO₂F₂, HF, NF₃, CF₄, BF₃, SiF₄, or the like. In terms of ease of handling, SF₆, NF₃, and CF₄ are preferable, and SF₆ is more preferable. Just one of these fluorine-containing inorganic compounds may be used, or two or more of these fluorine-containing inorganic compounds may be used together in a freely selected ratio.

### [Compound of formula 1]

The compound of formula 1 is a compound that is represented by formula 1: CH₃-R (R is a hydrogen atom, a chlorine atom, a bromine atom, an iodine atom, or an organic group other than a hydrocarbon group). The compound of formula 1 may be just one compound or may be two or more compounds used together in a freely selected ratio.

The "organic group other than a hydrocarbon group" referred to in the present specification is a functional group that includes at least one carbon atom (excluding functional groups formed of only carbon atoms and hydrogen atoms) or a functional group that includes one or more selected from oxygen, nitrogen, and sulfur and does not include a carbon atom. The organic group other than a hydrocarbon group may be an oxygen-containing organic group, a nitrogen-containing organic group, or a sulfur-containing organic group.

The oxygen-containing organic group may be a hydroxy (-OH), carboxy (-COOH), formyl (-CHO), formyloxy (-O-CH(=O)), acyl (-CR¹(=O)), acyloxy (-O-CR¹(=O)), alkoxy (-OR¹), alkoxycarbonyl (-C(=O)-OR¹), or the like, where R¹ is an alkyl, preferably a C1 to C4 alkyl, and more preferably a methyl or ethyl.

The nitrogen-containing organic group may be an unsubstituted amino (-NH₂), substituted amino (-NR²R³), nitro (-NO₂), cyano (-CN), or the like, where R² and R³ are independently a hydrogen or alkyl, at least one of R² and R³ is an alkyl, and the alkyl is preferably a C1 to C4 alkyl, and more preferably a methyl or ethyl.

The sulfur-containing organic group may be a mercapto (-SH), sulfo (-SO₃H), alkylthio (-SR⁴), or the like, where R⁴ is an alkyl, preferably a C1 to C4 alkyl, and more preferably a methyl.

R is preferably a hydrogen atom, chlorine atom, bromine atom, iodine atom, hydroxy (-OH), alkoxy (-OR¹), acyl (-CR¹(=O)), or substituted amino (-NR²R³) (R¹, R², and R³ are as previously described), and is more preferably a hydrogen atom, chlorine atom, hydroxy, methoxy, acetyl, or dimethylamino.

The compound of formula 1 may be CH₄, CH₃OH, CH₃Cl, CH₃Br, CH₃I, CH₃CHO, HCOOCH₃, CH₃COOCH₃, CH₃COOC₂H₅, CH₃NH₂, (CH₃)₂NH, (CH₃)₃N, CH₃CN, CH₃NO₂, CH₃SH, CH₃SCH₃, CH₃OCH₃, CH₃OC₂H₅, CH₃COCH₃, CH₃COC₂H₅, or the like, and, in terms of case of handling, is preferably CH₄, CH₃OH, CH₃Cl, CH₃COCH₃, CH₃OCH₃, or (CH₃)₃N, and more preferably CH₃OH.

### [Inert gas]

The inert gas may be N₂, He, Ne, Ar, Xe, Kr, CO, CO₂, or the like, is preferably N₂, Ar, He, CO, or CO₂, and is more preferably N₂ or Ar. The inert gas may be just one type of gas or may be two or more types of gases used together in a freely selected ratio.

### [Feedstock gas]

The feedstock gas contains the fluorine-containing inorganic compound, the compound of formula 1, and the inert gas. The fluorine-containing inorganic compound and the compound of formula 1 may each be a gas, a liquid, or a solid in a standard state (atmospheric pressure, 25°C), but are gases when the feedstock gas is caused to electrically discharge. A remaining portion of the feedstock gas other than the fluorine-containing inorganic compound, the compound of formula 1, and the inert gas is preferably impurities that are unavoidably mixed in from the surrounding environment.

The proportional contents of the fluorine-containing inorganic compound, the compound of formula 1, and the inert gas in the feedstock gas can be adjusted to any proportions without any specific limitations. The total proportional content of the compound of formula 1 and the fluorine-containing inorganic compound in the feedstock gas is preferably 0.1 volume% or more, more preferably 0.5 volume% or more, and even more preferably 1 volume% or more, and is preferably 95 volume% or less, more preferably 90 volume% or less, and even more preferably 85 volume% or less. In this case, the remaining portion of the feedstock gas is preferably the inert gas and impurities that are unavoidably mixed in from the surrounding environment.

The volume ratio of the fluorine-containing inorganic compound relative to the compound of formula 1 in the feedstock gas can be adjusted to any ratio without any specific limitations. The volume ratio of the fluorine-containing inorganic compound relative to the compound of formula 1 is preferably 0.8 or more in terms of inhibiting production of hydrocarbon side products, and is more preferably 1.8 or more for production of the target substance. The volume ratio can be 100 or less, and may be set as 25 or less, for example.

The feedstock gas contains the fluorine-containing inorganic compound, the compound of formula 1, and the inert gas when it is caused to electrically discharge. For example, in order to cause electrical discharge, a gas phase flow reactor having an electrical discharge mechanism (hereinafter, also referred to simply as a "gas phase flow reactor") may be supplied with the compound of formula 1, the fluorine-containing inorganic compound, and the inert gas as separately supplied gases so as to provide the feedstock gas, may be supplied with a gas obtained through premixing of all thereof so as to provide the feedstock gas, or may be supplied separately with a gas obtained by premixing a portion thereof and a gas of the remainder thereof so as to provide the feedstock gas.

In a case in which the compound of formula 1 or the fluorine-containing inorganic compound is a liquid having a sufficiently high vapor pressure that can easily be vaporized by heating or the like or is a gas in a standard state, the compound of formula 1 or the fluorine-containing inorganic compound can be supplied into the gas phase flow reactor as a gas without providing a separate vaporization chamber or the like. Control of the supply flow rate can be performed using a mass flow controller or the like.

In a case in which the compound of formula 1 or the fluorine-containing inorganic compound is a liquid having a low vapor pressure or is a solid in a standard state, the compound of formula 1 or the fluorine-containing inorganic compound can be vaporized in a separately provided vaporization chamber and then be supplied into the gas phase flow reactor. In the case of a solid, the solid can be introduced into the vaporization chamber after being converted to a liquid by heating.

For example, the compound of formula 1 or the fluorine-containing inorganic compound can be vaporized by introducing the compound of formula 1 or the fluorine-containing inorganic compound, in a liquid state, into a vaporization chamber held at a temperature and pressure at which sufficient vaporization of the compound of formula 1 or the fluorine-containing inorganic compound occurs. The temperature and pressure of the vaporization chamber are preferably held at a temperature and pressure that enable instantaneous vaporization of the compound of formula 1 or the fluorine-containing inorganic compound. The use of such a vaporization chamber makes it possible to continuously introduce the compound of formula 1 or the fluorine-containing inorganic compound into the vaporization chamber as a liquid, cause instantaneous vaporization thereof in the vaporization chamber, and then continuously supply the compound of formula 1 or the fluorine-containing inorganic compound into the gas phase flow reactor as a gas. Control of the supply flow rate can be performed by using a mass flow controller or the like to control gas that has been vaporized in the vaporization chamber or can be performed by using a liquid mass flow controller or the like to control continuous introduction of the compound of formula 1 or the fluorine-containing inorganic compound into the vaporization chamber in a liquid state. In a case in which both the compound of formula 1 and the fluorine-containing inorganic compound are to be vaporized, the compound of formula 1 and the fluorine-containing inorganic compound may be vaporized in separate vaporization chambers or may be vaporized in the same vaporization chamber. However, vaporization in separate vaporization chambers is preferable in terms of setting conditions of vaporization and controlling the supply flow rate. The compound of formula 1 and the fluorine-containing inorganic compound that have been vaporized may be diluted with the inert gas when they are introduced into the gas phase flow reactor.

The space velocity when the feedstock gas is caused to continuously flow in the gas phase flow reactor is not specifically limited but is preferably 0.01 h⁻¹ or more, more preferably 0.1 h⁻¹ or more, and even more preferably 0.3 h⁻¹ or more, and is preferably 100,000 h⁻¹ or less, more preferably 50,000 h⁻¹ or less, and even more preferably 10,000 h⁻¹ or less. A space velocity that is within any of the ranges set forth above makes it possible to avoid complication of electrical discharge and enables efficient production of the target substance without reduction of productivity.

### [Electrical discharge]

By causing electrical discharge of the feedstock gas in the gas phase flow reactor, various active species can be generated from the fluorine-containing inorganic compound and the compound of formula 1 that are contained in the feedstock gas. The compound of formula 1 is advantageous because it is particularly easy for a CH₃ radical that serves as a precursor for monofluoromethane to be generated from the compound of formula 1 during electrical discharge.

The method of electrical discharge of the feedstock gas can be a method that includes electrodes for applying a voltage that causes electrical discharge to occur. Examples of methods that can be used include high-frequency discharge, microwave discharge, dielectric barrier discharge, glow discharge, arc discharge, and corona discharge. High-frequency discharge, glow discharge, and arc discharge are preferable in terms of stability of electrical discharge and quantity of processed gas.

Although no specific limitations are placed on the pressure (absolute pressure) during electrical discharge so long as it is a pressure at which electrical discharge of the feedstock gas can occur in the adopted electrical discharge method, the pressure is preferably 1 PaA or higher, and more preferably 5 PaA or higher, and is preferably 1 MPaA or lower, and more preferably 0.5 MPaA or lower. A pressure (absolute pressure) that is within any of the ranges set forth above enables efficient production of the target substance.

### [Target substance]

The feedstock gas that has undergone electrical discharge is continuously released from the electrical discharge zone to thereby cause recombination of generated active species and produce monofluoromethane that is a target substance. The continuous release can be performed with a space velocity corresponding to the continuous flow of the feedstock gas.

The electrical discharge zone is a space in which electrical discharge of the feedstock gas is caused to occur. For example, in the case of a gas phase flow reactor including parallel plate electrodes as an electrical discharge mechanism, the electrical discharge zone is a space where electrical discharge occurs between the electrodes. Moreover, release to outside of the electrical discharge zone means exiting from the aforementioned space to outside of the space.

Note that after gas that has undergone electrical discharge has been released from the electrical discharge zone and has exited the gas phase flow reactor, the gas may be introduced into a heat exchanger and may be cooled. The mechanism of the heat exchanger is not specifically limited and may be air cooling, water cooling, or the like. Since the released material may contain hydrocarbons and the like other than the target substance, the target substance may be separated and purified through an optionally performed separation and purification step. Examples of separation and purification methods that can be used include distillation, absorption by a solution or the like, and membrane separation.

### EXAMPLES

The present disclosure is described in more detail below through examples. However, the present disclosure is not limited by these examples.

### (Example 1)

CH₃OH (vapor) as a compound of formula 1, SF₆ as a fluorine-containing inorganic compound, and Ar as an inert gas were introduced at flow rates of 10 sccm, 5 sccm, and 285 sccm, respectively, into a gas phase flow reaction tube made of metal in which parallel plate electrodes capable of high-frequency discharge were installed (frequency: 60 MHz, capacity: 35 L).

Electrical discharge was caused to occur through 500 W of supplied electrical power while holding a mixed gas of the compound of formula 1, the fluorine-containing inorganic compound, and the inert gas at 10 PaA (absolute pressure) inside the reaction tube. Gas was continuously released from the reaction tube and was trapped in an aluminum bag. A neutralization tube filled with KOH pellets was installed before the aluminum bag such that the trapped gas was gas that had passed through the neutralization tube.

The trapped gas was analyzed by gas chromatography-mass spectrometry (GC-MS) (Agilent 7890A produced by Agilent Technologies, Inc.) and flame ionization detection gas chromatography (GC-FID) (Agilent 6890N produced by Agilent Technologies, Inc.). The molar conversion rate of the compound of formula 1 was determined from area values for components in GC-FID and GC-MS that were obtained through the analysis, and this molar conversion rate was taken to be the feedstock conversion rate. In addition, the molar selectivity of CH₃F (monofluoromethane) in the product was determined from the aforementioned area values. The results are shown in Table 1.

### (Example 2)

Example 2 is the same as Example 1 with the exception that the flow rates of CH₃OH, SF₆, and Ar were changed to 10 sccm, 20 sccm, and 270 sccm, respectively. The results are shown in Table 1.

### (Example 3)

Example 3 is the same as Example 1 with the exception that the flow rates of CH₃OH, SF₆, and Ar were changed to 10 sccm, 30 sccm, and 260 sccm, respectively. The results are shown in Table 1.

### (Example 4)

Example 4 is the same as Example 1 with the exception that the flow rates of CH₃OH, SF₆, and Ar were changed to 10 sccm, 50 sccm, and 240 sccm, respectively. The results are shown in Table 1.

### (Example 5)

Example 5 is the same as Example 1 with the exception that the flow rates of CH₃OH, SF₆, and Ar were changed to 10 sccm, 240 sccm, and 50 sccm, respectively. The results are shown in Table 1.

### (Example 6)

Example 6 is the same as Example 3 with the exception that the inert gas was changed from Ar to N₂. The results are shown in Table 1.

### (Example 7)

Example 7 is the same as Example 1 with the exception that CH₄ was used as the compound of formula 1 and that the flow rates of CH₄, SF₆, and Ar were changed to 10 sccm, 30 sccm, and 260 sccm, respectively. The results are shown in Table 1.

### (Example 8)

Example 8 is the same as Example 7 with the exception that the flow rates of CH₄, SF₆, and Ar were changed to 10 sccm, 50 sccm, and 240 sccm, respectively. The results are shown in Table 1.

### (Example 9)

Example 9 is the same as Example 7 with the exception that the inert gas was changed from Ar to N₂. The results are shown in Table 1.

### (Example 10)

Example 10 is the same as Example 1 with the exception that CH₃OH was used as the compound of formula 1, CF₄ was used as the fluorine-containing inorganic compound, and the flow rates of CH₃OH, CF₄, and Ar were changed to 10 sccm, 10 sccm, and 280 sccm, respectively. The results are shown in Table 1.

### (Example 11)

Example 11 is the same as Example 1 with the exception that CH₃COCH₃ was used as the compound of formula 1 and that the flow rates of CH₃COCH₃, SF₆, and Ar were changed to 10 sccm, 50 sccm, and 240 sccm, respectively. The results are shown in Table 1.

### (Example 12)

Example 12 is the same as Example 1 with the exception that CH₃OCH₃ was used as the compound of formula 1 and that the flow rates of CH₃OCH₃, SF₆, and Ar were changed to 10 sccm, 50 sccm, and 240 sccm, respectively. The results are shown in Table 1.

### (Example 13)

Example 13 is the same as Example 1 with the exception that CH₃Cl was used as the compound of formula 1 and that the flow rates of CH₃Cl, SF₆, and Ar were changed to 10 sccm, 50 sccm, and 240 sccm, respectively. The results are shown in Table 1.

**Table 1**

| Examples | Compound of formula 1 | Flow rate [sccm] | Fluorine-containing inorganic compound | Flow rate [sccm] | Inert gas | Flow rate [sccm] | Proportional content of compound of formula 1 and fluorine-containing inorganic compound in feedstock gas [volume%] | Volume ratio of fluorine-containing inorganic compound relative to compound of formula 1 | Space velocity [h⁻¹] | Feedstock conversion rate [mol%] | CH₃F selectivity [mol%] | Hydrocarbon selectivity [mol%] |
|---|---|---|---|---|---|---|---|---|---|---|---|---|
| Example 1 | CH₃OH | 10 | SF₆ | 5 | Ar | 285 | 5% | 0.5 | 0.5 | 75.1% | 10.1% | 42.4% |
| Example 2 | | 10 | | 20 | | 270 | 10% | 2.0 | 0.5 | 72.5% | 62.6% | 0.9% |
| Example 3 | | 10 | | 30 | | 260 | 13% | 3.0 | 0.5 | 78.9% | 81.7% | 0.4% |
| Example 4 | | 10 | | 50 | | 240 | 20% | 5.0 | 0.5 | 82.0% | 87.5% | 0.7% |
| Example 5 | | 10 | | 240 | | 50 | 83% | 24.0 | 0.5 | 76.4% | 99.2% | 0.8% |
| Example 6 | | 10 | | 30 | N₂ | 260 | 13% | 3.0 | 0.5 | 79.2% | 62.1% | 0.8% |
| Example 7 | CH₄ | 10 | SF₆ | 30 | Ar | 260 | 13% | 3.0 | 0.5 | 72.1% | 37.3% | - |
| Example 8 | | 10 | | 50 | | 240 | 20% | 5.0 | 0.5 | 80.4% | 68.4% | - |
| Example 9 | | 10 | | 30 | N₂ | 260 | 13% | 3.0 | 0.5 | 76.7% | 10.8% | - |
| Example 10 | CH₃OH | 10 | CF₄ | 10 | Ar | 280 | 7% | 1.0 | 0.5 | 84.0% | 5.2% | 8.0% |
| Example 11 | CH₃COCH₃ | 10 | SF₆ | 50 | | 240 | 20% | 5.0 | 0.5 | 70.0% | 20.6% | 2.5% |
| Example 12 | CH₃OCH₃ | 10 | | 50 | | 240 | 2 0% | 5.0 | 0.5 | 72.1% | 25.4% | 1.6% |
| Example 13 | CH₃Cl | 10 | | 50 | | 240 | 20% | 5.0 | 0.5 | 32.7% | 28.0% | 1.3% |

It can be seen from Table 1 that mono fluoromethane (CH₃F) could be produced without using a catalyst in the examples. It can also be seen that production of hydrocarbon side products was sufficiently inhibited in Examples 2 to 13, in particular, in which the volume ratio of the fluorine-containing inorganic compound relative to the compound of formula 1 was 0.8 or more. It can also be seen through comparison with Example 1 that Examples 2 to 5 in which the volume ratio of the fluorine-containing inorganic compound relative to the compound of formula 1 was 1.8 or more were advantageous in the production of monofluoromethane (CH₃F).

### INDUSTRIAL APPLICABILITY

According to the present disclosure, it is possible to produce mono fluoromethane by a gas phase flow method without using a catalyst. The presently disclosed production method makes it possible to avoid reduction of yield caused by reduction of catalyst activity, enables continuous production of monofluoromethane, and has high industrial applicability.

## Claims

1. A method of producing monofluoromethane comprising causing electrical discharge of a feedstock gas containing: a fluorine-containing inorganic compound; a compound represented by formula 1: CH₃-R, where R is a hydrogen atom, a chlorine atom, a bromine atom, an iodine atom, or an organic group other than a hydrocarbon group; and an inert gas, while in a continuous flow state, and then causing continuous release to outside of an electrical discharge zone.

2. The method of producing monofluoromethane according to claim 1, wherein the fluorine-containing inorganic compound is one or more selected from the group consisting of SF₄, SF₆, SOF₂, SO₂F₂, HF, NF₃, CF₄, BF₃, and SiF₄.

3. The method of producing monofluoromethane according to claim 1 or 2, wherein the inert gas is one or more selected from the group consisting of N₂ and Ar.

4. The method of producing monofluoromethane according to any one of claims 1 to 3, wherein total proportional content of the fluorine-containing inorganic compound and the compound represented by formula 1 in the feedstock gas is not less than 1 volume% and not more than 85 volume%.

5. The method of producing monofluoromethane according to any one of claims 1 to 4, wherein a volume ratio of the fluorine-containing inorganic compound relative to the compound represented by formula 1 is 0.8 or more.

6. The method of producing monofluoromethane according to any one of claims 1 to 5, wherein a volume ratio of the fluorine-containing inorganic compound relative to the compound represented by formula 1 is 1.8 or more.
